## Europäisches Patentamt

## European Patent Office

## Office européen des brevets

(19)

(11) Veröffentlichungsnummer: **0 003 985**

**A1**

(12)

# EUROPÄISCHE PATENTANMELDUNG

(21) Anmeldenummer: 79100472.4

(22) Anmeldetag: 19.02.79

(51) Int. Cl.²: **C 07 C 27/20**
**B 01 J 1/00**

(30) Priorität: 11.03.78 DE 2810644

(43) Veröffentlichungstag der Anmeldung:
19.09.79 Patentblatt 79/19

(84) Benannte Vertragsstaaten:
BE DE FR GB IT NL SE

(71) Anmelder: BASF Aktiengesellschaft
Carl-Bosch-Strasse 38
D-6700 Ludwigshafen(DE)

(72) Erfinder: Elliehausen, Heinrich, Dr.
Bruehlstrasse 28
D-6700 Ludwigshafen(DE)

(72) Erfinder: Hohenschutz, Heinz, Dr.
Leibnizstrasse 12
D-6800 Mannheim 1(DE)

(72) Erfinder: Strohmeyer, Max, Dr.
Woogstrasse 41
D-6703 Limburgerhof(DE)

(72) Erfinder: Wolf, Dieter, Dr.
Auf der Wart 11
D-6718 Gruenstadt 1(DE)

(72) Erfinder: Zehner, Peter, Dr.
Alwin-Mittasch-Platz 11
D-6700 Ludwigshafen(DE)

(54) Verfahren zur Hydroformylierung von Olefinen.

(57) Hydroformylierung von Olefinen mit Kohlenmonoxid und Wasserstoff bei 10 bis 325 bar und 100 bis 170°C in Gegenwart von Kobalt- oder Rhodiumcarbonylkomplexen zu den entsprechenden, um ein Kohlenstoffatom reicheren Aldehyden und Alkoholen unter Verwendung von senkrecht stehenden zylindrischen Reaktoren, in denen ein koaxialer zylindrischer Einbau für einen Zwangsumlauf des Reaktionsgutes sorgt, indem man das Reaktionsgut im unteren Bereich des Reaktors entnimmt.

Croydon Printing Company Ltd.

BASF Aktiengesellschaft                    O. Z. 0050/033059

## Verfahren zur Hydroformylierung von Olefinen

Die vorliegende Erfindung betrifft ein verbessertes Verfahren zur Hydroformylierung von Olefinen.

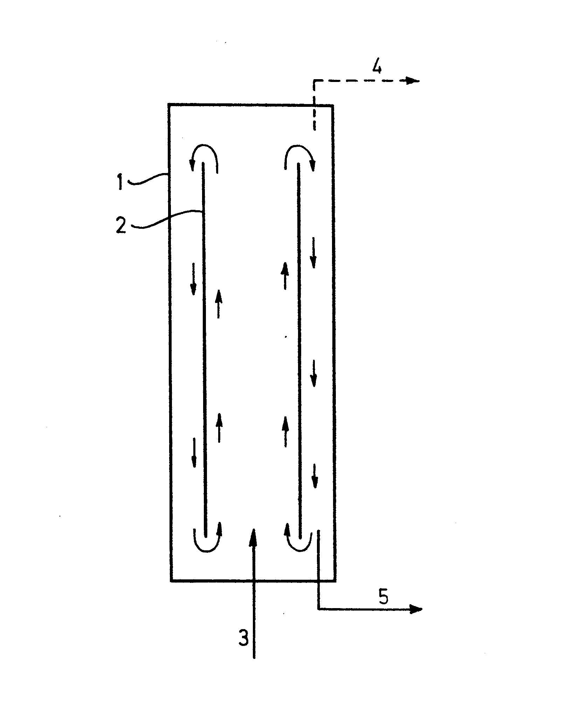

Die Hydroformylierung von Olefinen mit Kohlenmonoxid und Wasserstoff bei 10 bis 325 bar und 100 bis 170°C in Gegenwart von Kobalt- oder Rhodiumcarbonylkomplexen zu den entsprechenden, um ein Kohlenstoffatom reicheren Aldehyden und - bei Wasserstoffüberschuß - Alkoholen ist eine allgemein bekannte, in zahlreichen Varianten großtechnisch ausgeübte Reaktion. Wie ebenfalls allgemein bekannt ist, nimmt man die Umsetzung meistens in zylindrischen, senkrecht stehenden Hochdruckreaktoren (1) vor (vgl. die Zeichnung), in denen ein koaxialer zylindrischer Einbau (2), durch den das Reaktorvolumen etwa halbiert wird, für einen Zwangsumlauf des Reaktionsgutes sorgt. Die Einsatzstoffe - Olefin, Synthesegas und Katalysator - werden hierbei unten zugeführt (3), während die Reaktionsprodukte, mit denen der Reaktor beim stationären Betrieb gänzlich gefüllt ist, oben (4) entnommen werden. Ferner enthält der Reaktor sonstige Vorrichtungen wie Kühlrohre und Einbauten zur Erzeugung einer turbulenten Reaktionszone im inneren Zylinderraum, auf die es jedoch im vorliegenden Zusammenhang nicht ankommt.

Mi/Fe

Nachteilig bei dieser Arbeitsweise ist, daß lediglich ein Umsatz von 80 bis 96 %, bezogen auf das Olefin, erzielt werden kann. Besonders bei der Hydroformylierung von Olefinen, die bei Normaldruck gasförmig sind, beeinträchtigt dieser Umstand die Wirtschaftlichkeit des Verfahrens ganz erheblich, denn bei der Entspannung des Reaktionsgutes entweicht das nicht umgesetzte Olefin in die Abgase, aus denen es nur mit so großem Aufwand wieder gewonnen werden kann, daß man in der Regel die Verbrennung des Olefins vorzieht.

Die naheliegende Maßnahme, den Umsatz durch Verlängerung der Verweilzeit zu erhöhen, indem man den Reaktionsdurchsatz drosselt, liefert keine befriedigende Lösung des Problems, weil hierbei die Produktionskapazität vermindert wird, und weil bei einer derartigen Erhöhung der Gesamtverweilzeit aller Reaktionspartner die Bildung von unerwünschten Nebenprodukten begünstigt wird. Auch die Erhöhung der Temperatur, des Druckes und der Katalysatorkonzentration eignen sich nicht zur Behebung des geschilderten Nachteils, sei es, daß hierdurch das Verhältnis von n-Aldehyden bzw. n-Alkoholen zu den entsprechenden iso-Verbindungen verschlechtert wird, oder sei es, daß sich das Verfahren so verteuert, daß der Vorteil der Umsatzerhöhung hierdurch wieder aufgehoben wird.

Der Erfindung lag daher die Aufgabe zugrunde, den Umsatz bei der Hydroformylierung von Olefinen zu erhöhen, ohne dabei verfahrenstechnische oder wirtschaftliche Nachteile in Kauf nehmen zu müssen.

Es wurde ein verbessertes Verfahren zur Hydroformylierung von Olefinen mit Kohlenmonoxid und Wasserstoff bei 10 bis 325 bar und 100 bis 170°C in Gegenwart von Kobalt- oder Rhodiumcarbonylkomplexen zu den entsprechenden, um ein Kohlenstoffatom reicheren Aldehyden und Alkoholen unter Ver-

wendung von senkrechtstehenden zylindrischen Reaktoren (1), in denen ein koaxialer zylindrischer Einbau (2) für einen Zwangsumlauf des Reaktionsgutes sorgt, gefunden, welches dadurch gekennzeichnet ist, daß man das Reaktionsgut im unteren Bereich (5) des Reaktors entnimmt.

Da die Reaktorräume mit Aufwärts- und Abwärtsströmung zweckmäßigerweise das gleiche Volumen haben, wird durch die erfindungsgemäße Maßnahme nahezu eine Verdoppelung der Verweilzeit bewirkt, ohne daß sich an den sonstigen Reaktionsbedingungen, verglichen mit dem herkömmlichen Reaktoraustrag, etwas ändert. Dies ist von besonderem Vorteil, da die sonstigen Reaktionsbedingungen meist das Optimum des Zusammenwirkens aller Verfahrensparameter beim herkömmlichen Betrieb sind, so daß es aufgrund der erfindungsgemäßen Maßnahme nicht erforderlich ist, diese Optimum neu zu ermitteln. In fast allen Fällen ermöglicht der Austrag im Bereich des Reaktorbodens eine Umsatzerhöhung um mindestens 3,5 %, bezogen auf den theoretischen Umsatz von 100 %. Es erübrigt sich daher, auf die Reaktionsbedingungen näher einzugehen, da sie zum allgemein bekannten Stand der Technik gehören.

Die Entnahmeleitung (5) wird zweckmäßigerweise durch den Reaktorboden geführt. Sie soll soweit in den Mantelraum des Reaktors hineinragen, daß eine Rückvermischung mit frischem Ausgangsmaterial von der Einlaßleitung (3) praktisch nicht stattfinden kann.

Den größten Vorteil bringt die Verfahrensverbesserung bei der Hydroformylierung von Äthylen, Propylen und den Butenen, da hier der Verlust an nicht umgesetzten Olefinen deutlich herabgesetzt wird bzw. die Kosten in deren Rückgewinnung reduziert werden.

## Beispiel 1

Ein senkrecht stehender zylindrischer Hochdruckreaktor von 18 m lichter Höhe und 1,2 m Innendurchmesser mit einem zylindrischen Einbau, der den Zwangsumlauf des Reaktionsgutes in der in der Zeichnung skizzierten Weise ermöglicht, wurde bei einem Druck von 270 bar und einer durchschnittlichen Reaktorinnentemperatur von 159°C stündlich von unten mit 6 950 kg Propylen, 5 050 kg Synthesegas (= 9 000 $Nm^3$ CO und $H_2$ im Molverhältnis 1 : 1,25) und 7,6 kg einer 1,1 gew.%-igen wäßrigen Kobaltacetatlösung beschickt. Das Produktgemisch wurde nach Maßgabe des Zulaufs am unteren Ende des Reaktor-Mantelraumes entnommen.

Die folgende Übersicht zeigt das Verfahrensergebnis sowie im Vergleich dazu das Ergebnis bei herkömmlicher Entnahme am oberen Reaktorende.

|  | Entnahme des Reaktionsgutes | |
|---|---|---|
|  | unten | oben |
| Gesamtmenge, kg | 12 000 | 12 000 |
| davon Gasphase, kg | 300 | 600 |
| davon nicht umges. Propylen, kg | 90 | 320 |
| in % des einges. Propylens | 1,3 | 4,6 |
| davon Flüssigphase, kg (Gew.%) | 11 700 (100  ) | 11 400 (100  ) |
| n-Butyraldehyd | 6 700 ( 57,2) | 6 750 ( 59,4) |
| n-Butanol | 1 220 ( 10,4) | 910 ( 8,0) |
| Σn-Verbindungen | 7 920 ( 67,6) | 7 660 ( 67,4) |
| iso-Butylraldehyd | 2 320 ( 19,8) | 2 380 ( 20,9) |
| iso-Butanol. | 690 ( 5,9) | 510 ( 4,5) |
| Σiso-Verbindungen | 3 010 ( 25,7) | 2 890 ( 25,4) |
| Butylformiate | 260 ( 2,2) | 270 ( 2,4) |
| Rückstand | 530 ( 4,5) | 560 ( 4,9) |
| Gew.-Verhältnis (≈ Mol-verhältnis) n-Verbb.: iso-Verbb. | 78 : 22 | 75 : 25 |

Beispiel 2

Der Reaktor des Beispiels 1 wurde bei 170 bar und 150°C stündlich mit 3 600 kg Isobuten, 1 670 kg Synthesegas (Molverhältnis $CO : H_2 = 1 : 1,25$) und 4,7 kg einer 1,1 gew. %-igen wäßrigen Kobaltacetatlösung beschickt. Die folgende Tabelle gibt eine Übersicht über die Verfahrensergebnisse analog Beispiel 1.

|  | Entnahme des Reaktionsgutes | |
| --- | --- | --- |
|  | unten | oben |
| Gesamtmenge, kg | 5 270 | 5 270 |
| davon Gasphase, kg | 370 | 770 |
| davon nicht umges. Isobuten, kg | 115 | 350 |
| in % des einges. Isobutens | 3,7 | 9,7 |
| davon Flüssigphase, kg (Gew.%) | 4 900 (100 ) | 4 500 (100 ) |
| 3-Methylbutanal | 3 610 ( 73,6) | 3 610 ( 80,3) |
| 3-Methylbutanol | 680 ( 13,9) | 410 ( 9,1) |
| 2,2-Dimethylpropanal) | 195 ( 4,0) | 180 ( 4,0) |
| tert.-Butanol | 100 ( 2,0) | 100 ( 4,0) |
| 2,2-Dimethylpropanal | 130 ( 2,6) | 65 ( 1,4) |
| Rückstand | 195 ( 3,9) | 135 ( 2,9) |

0003985

Patentanspruch

Verfahren zur Hydroformylierung von Olefinen mit Kohlenmonoxid und Wasserstoff bei 10 bis 325 bar und 100 bis 170°C in Gegenwart von Kobalt- oder Rhodiumcarbonylkomplexen zu den entsprechenden, um ein Kohlenstoffatom reicheren Aldehyden und Alkoholen unter Verwendung von senkrecht stehenden zylindrischen Reaktoren (1), in denen ein koaxialer zylindrischer Einbau (2) für einen Zwangsumlauf des Reaktionsgutes sorgt, dadurch gekennzeichnet, daß man das Reaktionsgut im unteren Bereich (5) des Reaktors entnimmt.

Zeichn.

$\frac{1}{1}$

Europäisches
Patentamt

EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | <u>DE – B – 1 205 514</u> (BASF)<br>* Spalte 3, Figur *<br><br>-- | 1 |
| A | <u>DE – A – 1 938 102</u> (BASF)<br>* Seiten 3-7; Figur *<br><br>-- | 1 |
| A | <u>DE – C – 1 172 674</u> (METALLGE-SELLSCHAFT)<br>* Figur 2 *<br><br>-- | 1 |
| A | <u>BE – A – 674 648</u> (BASF)<br>* Figur 2 *<br><br>---- | 1 |

### KLASSIFIKATION DER ANMELDUNG (Int.Cl.²)

C. 07 C 27/20
B 01 J 1/00

### RECHERCHIERTE SACHGEBIETE (Int. Cl.²)

C 07 C 27/20
B 01 J 1/00

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 29-03-1979 | VAN GEYT |

EPA form 1503.1   06.78